# EUROPÄISCHE PATENTSCHRIFT

(11) **EP 3 955 811 B1**
(45) Veröffentlichungstag und Bekanntmachung des Hinweises auf die Patenterteilung: **22.07.2026**
(21) Anmeldenummer: 20718525.7
(22) Anmeldetag: 02.04.2020
(51) Int. Cl.: A61B 5/259, A61B 5/296

(54) **ELEKTRODE**
ELECTRODE
ÉLECTRODE

(30) Priorität: 16.04.2019 AT 503442019
(43) Veröffentlichungstag der Anmeldung: 23.02.2022
(73) Patentinhaber: Leonhard Lang GmbH, 6020 Innsbruck (AT)
(72) Erfinder: KUPSA, Thomas, 6020 Innsbruck (AT)
(74) Vertreter: Torggler & Hofmann Patentanwälte - Innsbruck
(86) Internationale Anmeldenummer: PCT/AT2020/060137
(87) Internationale Veröffentlichungsnummer: WO 2020/210850

(56) Entgegenhaltungen:
- WO-A2-01/17423
- WO-A2-2018/071944
- US-A- 5 150 708
- US-A1- 2017 127 969

## Beschreibung

Die Erfindung betrifft eine Elektrode nach Oberbegriff des Anspruchs 1.

Derartige medizinische Hautelektroden können als Messelektroden eingesetzt werden, die elektrische Signale vom menschlichen Körper ableiten. Sie können aber auch als Therapieelektroden eingesetzt werden, um Ströme dem menschlichen Körper zuzuführen. Die Elektroden werden zu diesem Zweck auf die Haut aufgeklebt und verfügen auf ihrer Unterseite im Allgemeinen über ein elektrisch leitendes Gel oder ein anderes elektrisches Kontaktmedium, das galvanisch mit einem Anschlusselement der Elektrode in Kontakt steht. An diesem Anschlusselement kann ein elektrischer Signalleiter angeschlossen werden, über den Ströme aus der Elektrode abgeleitet oder der Elektrode zugeführt werden können.

Ein Typ von Elektroden weist an der der Haut abgewandten Oberseite ein vorstehendes elektrisch leitendes Anschlusselement mit einer meist im Wesentlichen kugelkopfförmigen Anschlussstelle auf, an die sich ein Hals anschließt.

Bei der bisherigen Konstruktion von Elektroden dieses Typs ist das Anschlusselement zweiteilig ausgeführt. Der obere Teil (Oberknopf, Stud) dient als Kontakt- und Ankerelement für handelsübliche Signalleiter, beispielsweise EKG-Leitungen. Im Wesentlichen unterhalb des Trägers, also auf der der Haut zugewandten Seite, ist ein Unterknopf (Eyelet) der der Übernahme elektrischer Potenziale direkt vom Gel (Kontaktmedium) oder zur Übergabe an das Gel dient. Dabei ist das Eyelet sowohl elektrisch wie mechanisch mit dem Stud verbunden und zwar im Allgemeinen durch Vernieten der beiden Teile, derart, dass zwischen einem flanschartig seitlich abstehenden Haltebereich des Studs und einem ebensolchen Haltebereich des Eyelets das Trägermaterial der Elektrode fest eingeklemmt ist. Eine solche Konstruktion bietet einerseits einen guten mechanischen Halt des Anschlusselementes am Träger der Elektrode und erlaubt es andererseits, das Eyelet aus Materialien zu fertigen, die für eine Signalelektrode elektrische günstige Eigenschaften aufweisen, beispielsweise kann es dazu mit Silber beschichtet sein, wobei die Silberschicht wiederum ganzflächig oder zumindest in einem Teilbereich, der mit dem Gel in Kontakt steht, mit einer Schicht aus Silber / Silberchlorid (Ag /AgCl) überdeckt ist.

Die US 2017/0127969 A1 offenbart eine solche Elektrode.

Aus der anmeldereigenen Patentschrift WO 2018/071944 A2 ist es weiters bekannt, anstatt einer häufig üblichen zweiteilige Konstruktion, bei der das Anschlusselement aus zwei miteinander vernieteten Teilen (Stud und Eyelet) besteht, als Anschlusselement nunmehr einen einzigen Teil vorzusehen, der einerseits die Anschlussstelle zum lösbaren Anschließen eines Signalleiters bereitstellt und andererseits mit dem elektrischen Querleiter (vorzugsweise galvanisch) in Verbindung steht.

Auch die Patentschriften WO 01/17423 A2 und US 5,150,708 A offenbaren Elektroden mit solchen einteilig ausgebildeten Anschlusselementen.

Die Elektroden nach dem Stand der Technik sind allerdings teuer - wobei bei derartigen Massenartikeln bereits geringfügige Preisunterschiede ins Gewicht fallen.

Weiters steht die Schicht aus beispielsweise Silber / Silberchlorid (Ag /AgCl) bei Elektroden nach dem Stand der Technik vollflächig mit dem Kontaktmedium in Kontakt. Dies führt dazu, dass von Beginn an (erstes in Kontakt kommen des Kontaktmediums mit der Silber / Silberchlorid-Schicht) die Silber / Silberchlorid-Schicht durch das Kontaktmedium angegriffen wird. Es wird also an der gesamten Fläche der Silber / Silberchlorid-Schicht das Silber durch das Kontaktmedium in Silberchlorid umgewandelt. Es muss deshalb eine verhältnismäßig große Menge an Silber vorgesehen sein, um die Funktionsfähigkeit der Elektrode zu garantieren. Dies trägt wiederum zu den hohen Kosten bei Elektroden nach dem Stand der Technik bei.

Aufgabe der Erfindung ist es daher, eine verbesserte Elektrode, welche insbesondere oben genannte Probleme vermeidet, sowie ein Verfahren zur Herstellung einer solchen Elektrode anzugeben.

Erfindungsgemäß wird dies durch eine Elektrode gemäß Anspruch 1 und einem Verfahren gemäß Anspruch 13 erreicht.

Dadurch ist es möglich, die teure und aufwändige Beschichtung des gesamten Eyelets (Abschlusselementes) mit beispielsweise Silber/Silberchlorid durch den wesentlich kostengünstigeren und einfach zu fertigenden Leiter zu ersetzen.

Erfindungsgemäß wird vorgesehen, dass die dem Kontaktmedium zugewandte Seite des Leiters teilweise oder vollständig von dem Anschlusselement verdeckt wird.

Dadurch steht nur ein kleiner Teil bzw. nur die Randschicht der Silber / Silberchlorid-Schicht mit dem Kontaktmedium in Kontakt. Dies hat zur Folge, dass nur dieser kleine Teil bzw. nur die Randschicht der Silber / Silberchlorid-Schicht von dem Kontaktmedium angegriffen und somit in gleicher Zeit weniger Silber in Silberchlorid umgewandelt werden kann. Die Umwandlung findet als nur langsam von dem in Kontakt stehenden Bereichen der Silber/Silberchlorid-Schicht zu den verdeckten Bereichen der Silber/Silberchlorid-Schicht statt. Es ist deshalb möglich, die Menge an Silber in der Silber / Silberchlorid-Schicht zu reduzieren und somit weitere Kosten einzusparen.

Es kann vorgesehen sein, dass das Anschlusselement aus einem einzigen Teil besteht, das die Anschlussstelle zum lösbaren Anschließen einer Signalleitung aufweist.

Es kann aber auch vorgesehen sein, dass das Anschlusselement aus zumindest zwei Teilen besteht, wobei einer der beiden Teile die Anschlussstelle zum lösbaren Anschließen einer Signalleitung aufweist.

Das Anschlusselement selbst kann dabei aus mehreren Materialien bestehen, zum Beispiel aus vernickeltem Messing oder einem mit leitfähigem Material (insbesondere Kohlenstofffasern) dotiertem Kunststoff.

Besonders bevorzugt ist eine Ausbildung des Anschlusselementes, bei dem dieses derart ausgebildet ist, dass das Anschlusselement einen im Wesentlichen kugelförmigen Kopf, einen daran anschließenden, im Durchmesser reduzierten Hals, einen an das Ende des Halses anschließenden flanschförmig seitlich abstehenden Haltebereich und zumindest einen an den Haltebereich anschließenden Vorsprung aufweist.

Bei einem einteiligen Anschlusselement wird der Vorsprung (vorzugsweise ohne seitliche Berührung) durch eine Öffnung im Träger geführt, während der flanschförmig seitlich abstehende Haltebereich auf der Oberseite des Trägers aufliegt. Der im Durchmesser erweiterte, flanschförmig seitlich abstehende Haltebereich hält das Anschlusselement auch bei hohen Druckbelastungen sicher am Trägermaterial fest.

Der verformte, erweiterte Bereich des Vorsprungs des Haltelements liegt an der der Haut zugewandten Unterseite des Trägers bzw. an dem Leiter an und gewährleistet somit auch bei Druckbelastungen auf das Anschlusselement einen guten Halt des Anschlusselements am Träger.

Bei einem zweiteiligen Anschlusselement wird der Vorsprung (vorzugsweise ohne seitliche Berührung) durch eine Öffnung im Träger geführt, während der flanschförmig seitlich abstehende Haltebereich auf der Unterseite (oder Oberseite) des Trägers aufliegt. Der zweite Teil des Anschlusselements wird anschließend am Vorsprung angeordnet und liegt auf der Oberseite (oder Unterseite) des Trägers auf.

Eine weitere Ausführungsform der Erfindung sieht vor, dass der zumindest eine Vorsprung als sich in eine dem Haltebereich entgegengesetzte Richtung verjüngender Dorn ausgebildet ist. Dadurch ist es möglich, das Anschlusselement ohne vorherigem Herstellen einer durchgehenden Öffnung durch den Leiter und den Träger in den Träger bzw. Leiter einzubringen. Es wird also ein Arbeitsschritt eingespart.

An die elektrischen Eigenschaften des Anschlusselements sind beim Erfindungsgegenstand keine hohen Anforderungen gestellt. Er kann daher aus kostengünstigem Material, beispielsweise aus einem einfachen Metallblech bestehen. Das Anschlusselement braucht nämlich keine besonderen elektrischen Eigenschaften aufweisen, denn diese für Bioelektroden günstigen elektrischen Eigenschaften kann lediglich der Leiter aufweisen, der mit dem elektrischen Kontaktmedium in Verbindung steht.

Dieser Leiter kann dabei grundsätzlich jede beliebige Geometrie aufweisen, in bevorzugten Ausführungsbeispielen der Erfindung kann der Leiter jedoch als rotationssymmetrische oder im Wesentlichen quaderförmige Leiterscheibe ausgebildet sein. Diese Leiterscheibe kann dabei zumindest teilweise über den verformten, erweiterten Bereich vorstehen.

Um bei einer Elektrode ein geringes Rauschen und eine Depolarisation im Falle einer Defibrillation zu erreichen, werden aktuell Redoxpaare verwendet. Diese können oxidiert bzw. reduziert werden und nehmen dabei mindestens ein Elektron auf oder geben mindestens ein Elektron ab. Aktuell werden verschiedenste Substanzen für diese Depolarisation verwendet. Am häufigsten werden Silber / Silberchlorid und Zinn / Zinnchlorid verwendet. Für die vorliegende Erfindung sind allerdings sämtliche Redoxpaare denkbar, welche eine Depolarisation der Elektrode ermöglichen. Dabei können die Redoxpaare aktiv beigemengt oder durch Reaktionen möglicherweise in situ erzeugt werden.

Nachdem beispielsweise Silber / Silberchlorid eine relativ teure Substanz ist, reicht es aus, wenn gemäß einem weiteren Aspekt der Erfindung vorgesehen ist, dass der Leiter vorzugsweise einseitig mit einem elektrisch leitenden Material versehen ist, welches mit dem Anschlusselement und mit dem Kontaktmedium galvanisch verbunden ist.

Durch die Maßnahme, den Leiter vorzugsweise einseitig mit einem elektrisch leitenden Material zu versehen, kann man weiter Kosten sparen. Der eigentliche Leiter kann nämlich kostengünstige Materialien, wie beispielsweise Metall oder Kunststoff verwenden, während man an dem für die günstigen elektrischen Eigenschaften der Bioelektrode kritischen Übergangsbereich zum elektrischen Kontaktmedium (insbesondere Gel) ein zweites, elektrisch leitendes Material wie beispielsweise Silber / Silberchlorid verwenden kann. Es reicht aus, wenn dieses Material nur lokal in diesem Bereich vorhanden ist.

Insbesondere kann der Leiter aus einer mit einem elektrisch leitenden Material versehen Kunststofffolie bestehen.

Insgesamt liegt der Erfindung der Grundgedanke zu Grunde, das Anschlusselement für den Signalleiter so auszubilden, dass es gut in der Elektrode verankert ist, während es auf die elektrischen Eigenschaften weniger ankommt und damit kostengünstige Materialien verwendet werden können.

Andererseits können die für die günstige elektrische Signalleitung vorgesehenen teureren Materialen lediglich in dem elektrisch kritischen Bereich am Übergang zum elektrischen Kontaktmedium (Gel) verwendet werden. Diese Aufgabe übernimmt der Leiter. Ganz kurz formuliert würde man sagen, das elektrisch leitende Anschlusselement ist, abgesehen von der Grundeigenschaft der elektrischen Leitung hauptsächlich für die "Mechanik" verantwortlich. Beim Leiter ist es umgekehrt: er braucht keine besonderen mechanischen Eigenschaften zu erfüllen und lediglich im Bereich der Übergangsstelle zum elektrischen Kontaktmedium (Gel) aus dafür günstigen Materialien bestehen. Insofern ist der Leiter ohne besondere mechanische Aufgaben für die "Elektrik" zuständig.

Weitere Vorteile und Einzelheiten der Erfindung werden anhand der nachfolgenden Figurenbeschreibung näher erläutert. Darin zeigen:
- Fig. 1: eine schematische Unteransicht (später die der Haut zugewandte Seite) der Herstellungsschritte eines Ausführungsbeispiels einer erfindungsgemäßen Elektrode bis zur fertigen Elektrode,
- Fig. 2: eine schematische Draufsicht der Herstellungsschritte eines Ausführungsbeispiels einer erfindungsgemäßen Elektrode bis zur fertigen Elektrode, wobei nur ein Teil der Prozessschritte in einer Draufsicht dargestellt ist,
- Fig. 3: eine Abfolge der Schnitte gemäß der Linie A-A der Figur 1, wobei die Darstellung zur besseren Visualisierung als schematische Darstellung zu verstehen ist,
- Fig. 4: entfällt
- Fig. 5: entfällt
- Fig. 6: entfällt
- Fig. 7: eine schematische Unteransicht (später die der Haut zugewandte Seite) der Herstellungsschritte eines weiteren Ausführungsbeispiels einer erfindungsgemäßen Elektrode bis zur fertigen Elektrode,
- Fig. 8: eine schematische Draufsicht der Herstellungsschritte eines weiteren Ausführungsbeispiels einer erfindungsgemäßen Elektrode bis zur fertigen Elektrode, wobei nur ein Teil der Prozessschritte in einer Draufsicht dargestellt ist,
- Fig. 9: eine Abfolge der Schnitte gemäß der Linie A-A der Figur 7, wobei die Darstellung zur besseren Visualisierung als schematische Darstellung zu verstehen ist,
- Fig. 10: eine schematische Unteransicht eines Ausführungsbeispiels eines erfindungsgemäßen Trägers mit einer Klebstoffschicht,
- Fig. 11: eine schematische Unteransicht eines weiteren Ausführungsbeispiels eines erfindungsgemäßen Trägers mit einer Klebstoffschicht,
- Fig. 12a: eine schematische Seitenansicht eines Ausführungsbeispiels eines erfindungsgemäßen Anschlusselements,
- Fig. 12b: eine schematische Draufsicht eines Ausführungsbeispiels eines erfindungsgemäßen Anschlusselements,
- Fig. 13a: eine schematische Seitenansicht eines Verankerungsvorgangs eines erfindungsgemäßen Anschlusselements in einem Träger,
- Fig. 13b: eine schematische Draufsicht (später die der Haut abgewandte Seite) eines Verankerungsvorgangs eines erfindungsgemäßen Anschlusselements in einem Träger,
- Fig. 14a: eine schematische Ansicht eines weiteren Ausführungsbeispiels eines erfindungsgemäßen Anschlusselements,
- Fig. 14b: eine schematische Ansicht eines weiteren Ausführungsbeispiels eines erfindungsgemäßen Anschlusselements,
- Fig. 15a: eine schematische Seitenansicht eines weiteren Ausführungsbeispiels eines erfindungsgemäßen Anschlusselements,
- Fig. 15b: eine schematische Draufsicht eines weiteren Ausführungsbeispiels eines erfindungsgemäßen Anschlusselements,
- Fig. 16a: eine schematische Seitenansicht eines weiteren Ausführungsbeispiels eines erfindungsgemäßen Anschlusselements,
- Fig. 16b: eine schematische Draufsicht eines weiteren Ausführungsbeispiels eines erfindungsgemäßen Anschlusselements,
- Fig. 17a: eine schematische Seitenansicht eines weiteren Ausführungsbeispiels eines erfindungsgemäßen Anschlusselements,
- Fig. 17b: eine schematische Draufsicht eines weiteren Ausführungsbeispiels eines erfindungsgemäßen Anschlusselements,
- Fig. 18: eine Explosionsdarstellung eines weiteren Ausführungsbeispiels mit einem zweiteiligen Anschlusselement,
- Fig. 19: eine schematische Unteransicht (später die der Haut zugewandte Seite) der Herstellungsschritte eines Ausführungsbeispiels (zweiteiliges Anschlusselement) einer erfindungsgemäßen Elektrode bis zur fertigen Elektrode,
- Fig. 20: eine schematische Draufsicht der Herstellungsschritte eines Ausführungsbeispiels einer erfindungsgemäßen Elektrode bis zur fertigen Elektrode, wobei nur ein Teil der Prozessschritte in einer Draufsicht dargestellt ist, und
- Fig. 21: eine Abfolge der Schnitte gemäß der Linie A-A der Figur 18, wobei die Darstellung zur besseren Visualisierung als schematische Darstellung zu verstehen ist.

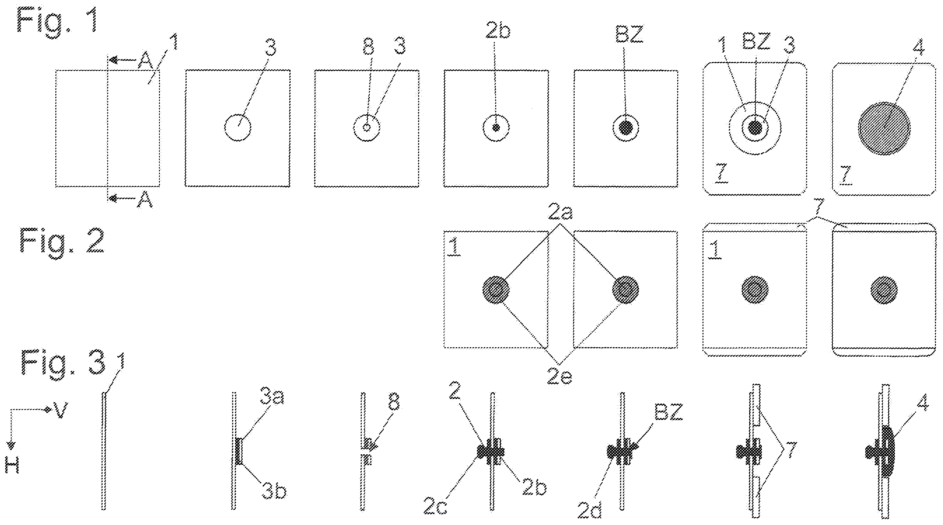

Unter Bezugnahme auf die Figuren 1 bis 3 wird nun der Verfahrensablauf zur Herstellung eines Ausführungsbeispiels einer erfindungsgemäßen Elektrode zum Anbringen auf der menschlichen Haut näher erläutert.

Ausgegangen wird von einem elektrisch nichtleitenden Träger 1. Das Trägermaterial dient zur Verankerung der elektrischen Komponenten der Elektrode. Es kann beispielsweise aus einer (flexiblen) Folie (beispielsweise aus PET oder TPU) bestehen, die auf der in der Zeichnung der Figur 1 nach oben weisenden Unterseite vollständig oder teilweise mit einem Klebstoff beschichtet ist, der beispielsweise selbstklebend (pressure sensitive adhesive) oder thermoaktivierbar (hot melt) ausgeführt sein kann.

Auf dieses Trägermaterial wird nun in einem nächsten Schritt ein rotationssymmetrischer Leiter 3 befestigt, insbesondere verklebt oder aufgedruckt. Der Leiter weist gemäß einer bevorzugten Variante der Erfindung zwei unterschiedlich elektrisch leitende Materialien oder ein elektrisch nichtleitendes Material 3b und ein elektrisch leitendes Material 3a auf, wobei das elektrisch leitende Material 3a bzw. eines der beiden elektrisch leitenden Materialien später mit dem elektrischen Anschlusselement 2 und mit dem Kontaktmedium 4 (Gel) galvanisch verbunden wird.

Bei dem dargestellten Ausführungsbeispiel handelt es sich um einen schwarz bzw. grau dargestellten, kreisförmigen Leiter 3 aus einer Kunststofffolie. Der Leiter 3 kann aber auch aus einem Metall oder einem leitfähigen mit Kohlenstofffasern dotiertem Kunststoff bestehen.

Im Bereich der späteren Kontaktstelle mit dem elektrischen Kontaktmedium 4 (Gel) ist dieser Leiter 3 mit einer Schicht 3a aus beispielsweise Silber / Silberchlorid oder Zinn / Zinnchlorid oder einem anderen Redoxpaar beschichtet.

In einem weiteren Schritt wird nun eine Öffnung 8 durch den elektrischen Leiter 3 und den Träger 1 vorgesehen. Das kann beispielsweise durch Ausstanzen geschehen. Anschließend folgt die Einbringung des Anschlusselementes 2, das einen Vorsprung 2b aufweist, welcher über die Unterseite des Trägers 1 und des Leiters 3 vorsteht.

Das Anschlusselement 2 weist beim dargestellten Ausführungsbeispiel im Anschluss an den im Wesentlichen kugelförmigen Kopf 2c einen im Durchmesser reduzierten Hals 2d auf, an dem sich ein flanschförmig seitlich abstehender Haltebereich 2e und ein Vorsprung 2b anschließt

Insgesamt ist der seitlich abstehende flanschförmige Haltebereich 2e im Wesentlichen tellerförmig ausgebildet. Er ist für die Verteilung und Übertragung von auf das Anschlusselement 2 aufgebrachten Druckkräften auf den Träger 1 zuständig.

Kommt ein Anschlusselement 2 zum Einsatz, das aus einem einzigen Teil besteht, das einerseits mit dem elektrischen Leiter 3 in Verbindung steht und andererseits die Anschlussstelle 2a zum lösbaren Anschließen eines (hier nicht dargestellten) Signalleiters aufweist, ist damit eine kostengünstige Herstellung der Elektrode möglich, weil das meist kostenintensive Eyelet (Unterknopf) entfallen kann. Für die mechanische Verankerung reicht die einteilige Ausbildung des Anschlusselementes aus.

Die Anforderungen an die elektrischen Eigenschaften sind gering. Damit können einfache Konstruktionen, wie beispielsweise ein tiefgezogenes Metallteil als Anschlusselement 2 verwendet werden. Die etwas diffizileren elektrischen Aufgaben übernimmt also hier nicht das sonst übliche Eyelet (Unterknopf) sondern der Leiter 3, der mit dem später aufgebrachten elektrischen Kontaktmedium 4 (Gel) in Verbindung steht.

Es erfolgt also eine Trennung der Aufgaben. Das elektrische Anschlusselement 2 ist abgesehen von der Basiseigenschaft elektrisch leitend zu sein, im Wesentlichen für den mechanischen Halt in der Elektrode verantwortlich, während der Leiter 3 von mechanischen Aufgaben weitgehend befreit ist. Das erlaubt es eine günstige Materialwahl zu treffen. Insbesondere ist es möglich, teurere - aus elektrischer Sicht günstige - Materialien nur dort (Stelle 3a) vorzusehen, wo später der Kontakt mit dem Gel erfolgt.

Das elektrisch leitende Anschlusselement 2 kann - wie bereits erwähnt - aus einem tiefgezogenen Metallblech bestehen. Es ist dann im Inneren zumindest teilweise hohl. Es kann aber auch aus einem leitfähigen Kunststoff bestehen, beispielsweise aus ABS, das mit leitfähigen Karbonfasern dotiert ist.

Günstigerweise wird man das Anschlusselement im Wesentlichen rotationssymmetrisch ausbilden. Andere Varianten sind auch möglich.

Um das elektrische Anschlusselement 2 endgültig in der Elektrode zu fixieren und insbesondere auch gegen Zugbelastungen zu sichern, wird in einem nächsten Schritt der Vorsprungs 2b derart verformt, dass ein verformter, erweiterter Bereich BZ hergestellt wird.

Die Verformung des Vorsprungs 2b kann dabei durch Aufschmelzen, Umbördeln, Aufspreizen oder Umbiegen erfolgen. Es ist aber auch jedes andere geeignete Verfahren anwendbar.

Durch die Verformung des Vorsprungs 2b wird über den verformten, erweiterten Bereich BZ eine galvanische Verbindung zwischen dem Anschlusselement 2 und dem leitenden Material 3a des Leiters 3 und andererseits mittels Form- und/oder Kraftschluss eine mechanische Befestigung des Anschlusselements 2 an dem Träger 1 hergestellt.

Auf die Unterseite des Trägers 1 wird nun eine Pflasterschicht 7 aufgebracht, insbesondere verklebt, wobei die Pflasterschicht vorzugsweise mittels einer patientenseitigen Beschichtung aus biokompatiblem Klebstoff auf die Haut aufklebbar ist, um die Elektrode zu fixieren.

Dabei ist es auch möglich, die Pflasterschicht über eine auf ihr aufgebrachte Schicht aus Selbstkleber oder aus einem thermoaktivierbaren Kleber mit dem Träger 1 zu verkleben.

Das Pflastermaterial dient letztlich dazu, die Elektrode auf der Patientenhaut zu fixieren. Geeignete Pflastermaterialien können beispielsweise aus einer Folie (beispielsweise PE), aus einem Schaumband (beispielsweise PE-Schaum) oder aus Vliesstoffen bestehen. Die Pflastermaterialien sind üblicherweise patientenseitig mit einem biokompatiblen Klebstoff beschichten.

In einem letzten Herstellungsschritt der Elektrode gemäß den Figuren 1 bis 3 erfolgt die Einbringung des elektrischen Kontaktmediums 4 in eine dafür vorgesehene Aussparung im Pflastermaterial 7. Das elektrische Kontaktmedium 4 ermöglicht die (vorzugsweise ionen-basierte) Leitung von körpergenerierten elektrischen Potenzialen oder von gerätegenerierten Mess- oder Stimulationsströmen von der Körperoberfläche (Haut) zum elektrischen Anschlusselement 2 und umgekehrt. Das Kontaktmedium 4 kann beispielsweise aus einem mit Chloriden dotiertem Gel bestehen, das entweder in mehr oder weniger flüssiger Form (mehr oder weniger geliert) oder als quervernetzte Polymer-Matrix (Hydrogel) vorliegt. Es ist aber auch möglich, das elektrische Kontaktmedium 4 mit anderen Mitteln zu erzeugen, beispielsweise als leitfähiger Kleber oder als mit Salzlösung gefüllter Schwamm.

Jedenfalls wird das elektrische Kontaktmedium 4, wie es der letzte Schritt in den Figuren 1 bis 3 zeigt, in die Aussparung im Pflastermaterial 7 eingebracht. Es kontaktiert darin das elektrisch leitende Material 3a (insbesondere Silber / Silberchlorid).

Das Zusammenwirken des elektrisch leitenden Materials 3a, insbesondere die Beschichtung mit Silber / Silberchlorid oder einem anderen geeigneten Material einerseits und dem Material des elektrisch leitenden Kontaktmediums 4 andererseits, erlaubt es günstige elektrische Eigenschaften der Elektrode, wie beispielsweise rauschfreie Signalübertragung oder depolarisierende Wirkungen zu erzielen, wobei der Einsatz des relativ teuren elektrisch leitenden Materials 3a des Leiters 3 auf jenen Bereich beschränkt bleiben kann, in dem der Kontakt mit dem Kontaktmedium 4 erfolgt. Das senkt die Kosten weiter.

Insgesamt ergibt sich bei der Herstellung gemäß den Figuren 1 bis 3 eine "zentrale" Elektrode, bei der das Anschlusselement 2 und das Kontaktmedium 4 (Gel) direkt übereinander angeordnet sind.

Die für das Ausführungsbeispiel gemäß den Figuren 1 bis 3 wesentlichen Verfahrensschritte sind die folgenden:
- Anordnen, vorzugsweise Verkleben oder Aufdrucken, eines Leiters (3) auf der der Haut zugewandten Unterseite eines elektrisch nichtleitenden Trägers (1),
- Einbringen eines Anschlusselementes (2) durch den Träger (1) hindurch, derart dass auf der Unterseite oder der Oberseite des Trägers (1) der Vorsprung (2b) des Anschlusselements (2) vorsteht und das Anschlusselement (2) - vorzugsweise mit einem seitlich abstehenden, tellerförmigen Haltebereich (2e) - an der Oberseite oder der Unterseite des Trägers (1) anliegt,
- Verankern des Anschlusselements (2) im Träger (1) derart, dass eine elektrisch leitfähige Verbindung zwischen dem Anschlusselement (2) und dem Leiter (3) und eine mechanische Befestigung des Anschlusselements (2) am Träger (1) hergestellt wird.

Schließlich werden dann zur Fertigstellung der Elektrode noch folgende Schritte gesetzt:
- Anbringen - vorzugsweise Verkleben - einer hautseitig klebenden Pflasterschicht 7 mit dem Träger 1,
- Einbringen von einem elektrischen Kontaktmedium 4 - vorzugsweise einem Gel - in eine Aussparung der Pflasterschicht 7, derart dass der darunterliegende Leiter 3 kontaktiert wird.

Der verformte, erweiterte Bereich BZ kann auch nicht kreisförmig, sondern lamellenartig ausgebildet ist. Der verformte, erweiterte Bereich BZ kann grundsätzlich jede beliebige Form aufweisen.

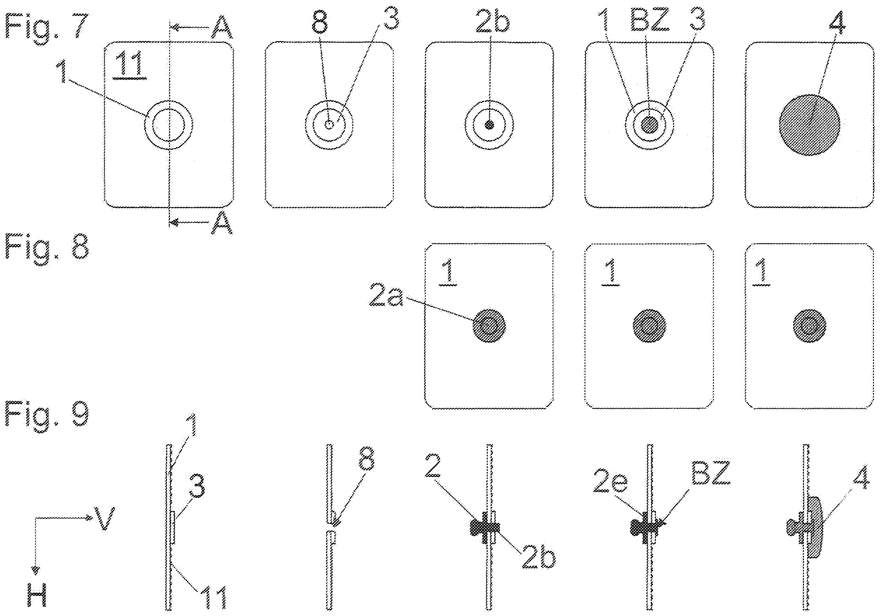

Bei dem in den Figuren 7 bis 9 dargestellten Ausführungsbeispiel stimmen die meisten Verfahrensschritte mit denen in Figur 1 bis 3 überein, weshalb auch gleiche Bezugszeichen gleiche Teile bezeichnen.

Der Unterschied besteht im Wesentlichen darin, dass auf dem Träger 1 eine biokompatible Klebstoffschicht 11 zum Aufkleben der Elektrode auf eine Haut eines Patienten vorgesehen ist. Somit kann die Pflasterschicht 7 entfallen und ein weiterer Prozessschritt eingespart werden.

Die Klebstoffschicht 11 kann dabei vor oder nach dem Aufbringen des Leiters 3 auf den Träger 1 aufgebracht werden bzw. ist die Klebstoffschicht 11 bereits am Ausgangsmaterial des Trägers 1 vorhanden.

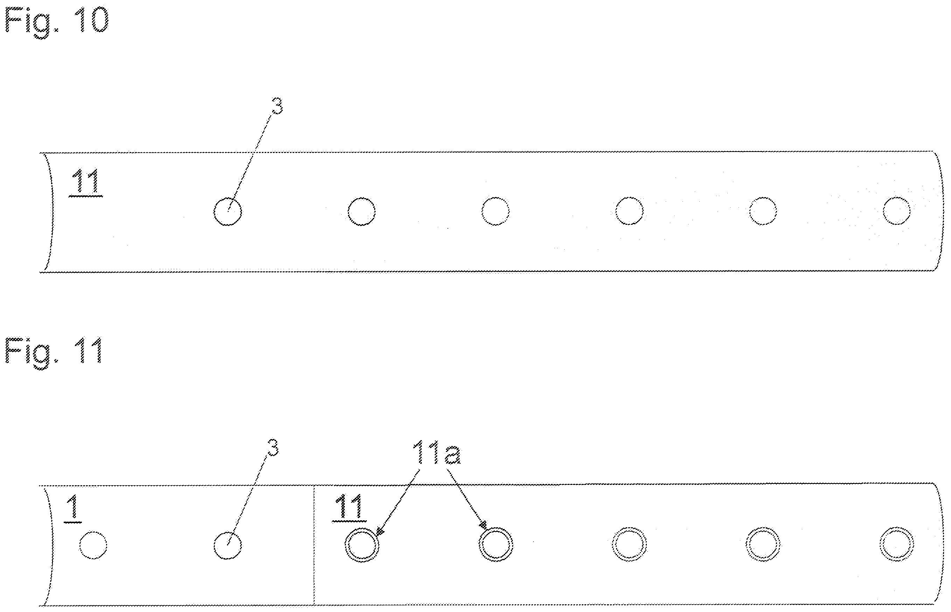

In den Figuren 10 und 11 sind die oben genannten Varianten zum Aufbringen des Klebstoffs 11 gezeigt.

In Figur 10 ist der Klebstoff 11 bereits am Träger 1 vorhanden bzw. wird vor dem Aufbringen des Leiters 3 aufgebracht. Der Leiter 3 wird dabei anschließend auf die Klebstoffschicht 11 aufgebracht. Dabei kann der Leiter 3 durch die Klebstoffschicht 11 gehalten werden, wodurch der Leiter 3 nicht zusätzlich mit dem Träger 1 verklebt werden muss.

In Figur 11 wird der Leiter 3 auf den Träger 1 aufgebracht und anschließend der Klebstoff 11 auf den Träger 1 aufgebracht. Dabei ist eine Ausnehmung 11a vorgesehen, damit der Leiter 3 nicht von dem Klebstoff 11 verdeckt wird.

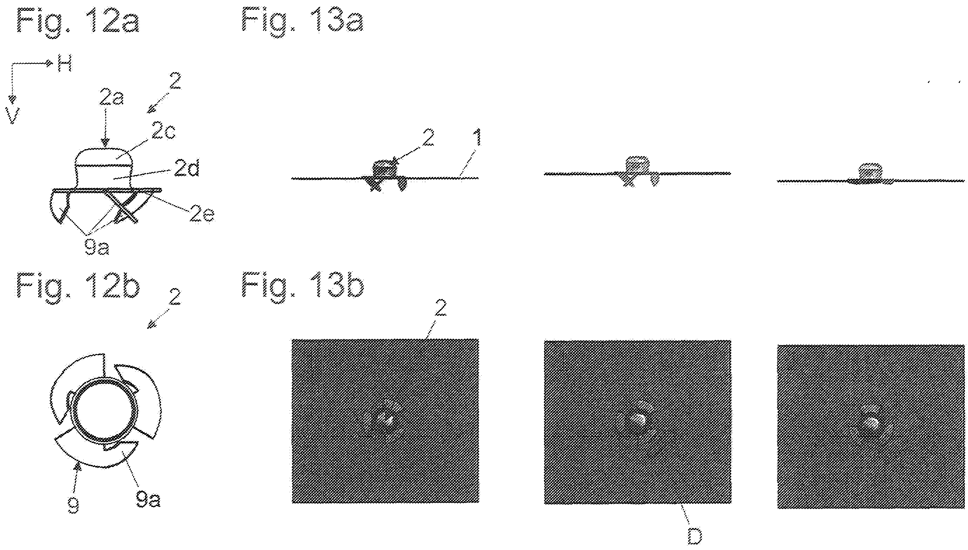

Die Figuren 12a und 12b zeigen ein Ausführungsbeispiel eines erfindungsgemäßen Anschlusselements 2. Es ist erkennbar, dass das Anschlusselement 2 Flügelsegmente 9 aufweist, welche sowohl den Vorsprung als auch den Haltebereich des Anschlusselements 2 bilden. Die gegenüber einer horizontalen Lage H geneigten Flügelsegmentabschnitte 9a können dabei gleich oder unterschiedlich lang ausgebildet sein. Es ist auch denkbar, dass die Flügelsegmente 9 zumindest abschnittsweise scharfkantig ausgebildet sind, um ein Durchdringen eines Trägers 1 und eines Leiters 3 zu erleichtern.

Die Figuren 13a und 13b zeigen schematische Ansichten eines Verankerungsvorgangs eines erfindungsgemäßen Anschlusselements in einem Träger, mit einem wie in den Figuren 12a und 12b dargestellten Anschlusselement 2.

In einem ersten Schritt wird dazu das Anschlusselement 2 von einer später der Haut abgewandten Oberseite eines Trägers 1 durch den Träger 1 und den auf der Unterseite des Trägers 1 angebrachten, nicht dargestellten Leiter 3 gedrückt. Das heißt, das Anschlusselement 2 durchdringt den Träger 1 und den Leiter 3 mit den Flügelsegmentabschnitten 9a.

In einem nächsten Schritt wird das Anschlusselement 2 in eine Richtung D verdreht. Dadurch erfolgt eine bessere Verankerung des Anschlusselements 2 im Träger 1.

In einem letzten Schritt werden die Flügelsegmentabschnitte 9a in Richtung der Unterseite des Trägers 1 über eine horizontale Lage H hinaus aufgebogen, wodurch eine Klemmung des Trägers 1 und des Leiters 3 erreicht wird. Somit ist auch eine elektrische Verbindung des Anschlusselements 2 mit dem Leiter 3 und eine mechanische Befestigung des Anschlusselements 2 am Träger 1 sichergestellt. Es ist allerdings auch möglich, dass die Flügelsegmentabschnitte nur aufgebogen werden, bis sie sich in einer horizontalen Lage H befinden.

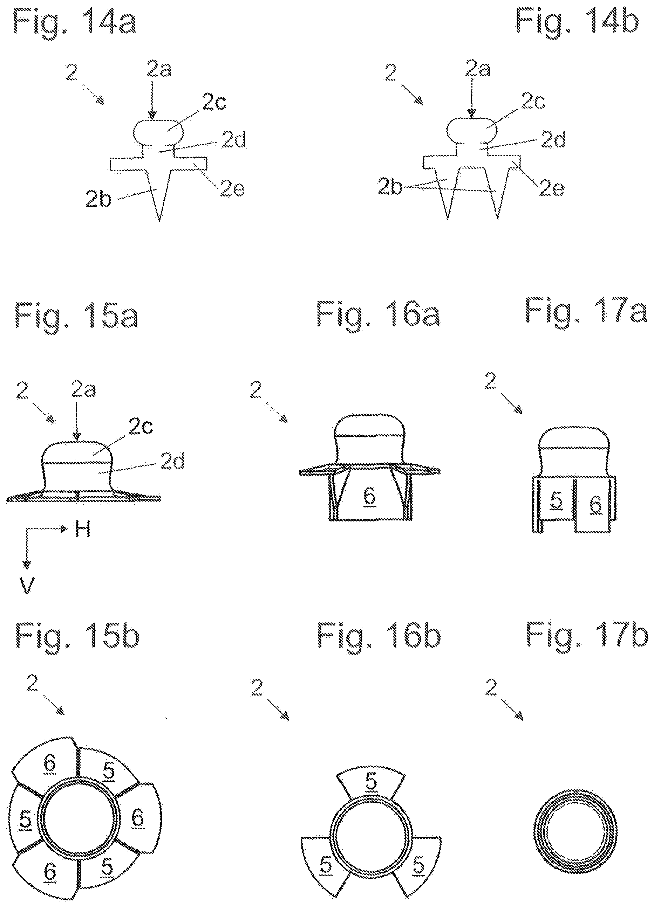

Die Figur 14a zeigt ein Ausführungsbeispiel eines Anschlusselements 2 bei welchem der Vorsprung 2b als sich verjüngender Dorn ausgebildet ist. Dadurch ist es möglich, das Anschlusselement 2 ohne vorheriges Herstellen einer durchgehenden Öffnung 8 durch den Leiter 3 und den Träger 1 in den Träger 1 bzw. Leiter 3 einzubringen. Es wird also ein Arbeitsschritt eingespart.

Figur 14b zeigt ein Ausführungsbeispiel eines Anschlusselements 2, bei welchem zwei Vorsprünge 2b als sich verjüngende Dorne ausgebildet sind. Es kann allerdings eine beliebige Anzahl an Vorsprüngen 2b vorgesehen sein. Außerdem können auch mehrere Vorsprünge 2b, welche nicht dornförmig ausgebildet sind, vorgesehen sein. Die Mehrzahl an Vorsprüngen 2b kann dabei rotationssymmetrisch oder nicht rotationssymmetrisch am Anschlusselement 2 angeordnet sein.

Die Figuren 15a bis 17b zeigen Ausführungsbeispiele eines Anschlusselements 2, bei welchem der Vorsprung und der flanschartige Haltebereich des Anschlusselements 2 aus zumindest einem ersten Segment 5 und zumindest einem zweiten Segment 6 gebildet werden.

Es ist auch ersichtlich, dass die zweiten Segmente 6 länger als die ersten Segmente 5 ausgebildet sind. Die Segmente 5, 6 können auch gleich lang ausgebildet sein, oder die Segmente 5 können länger als die Segmente 6 ausgebildet sein.

Figur 15a zeigt das Anschlusselement in einer Frontalansicht, wenn sich alle Segmente 5, 6 in einer Horizontalen Lage H befinden. Figur 15b zeigt die entsprechende Draufsicht.

Figur 16a zeigt das Anschlusselement in einer Frontalansicht, wenn sich die Segmente 5 in einer horizontalen Lage H und die Segmente 6 sich in einer vertikalen Lage V befinden. Figur 16b zeigt die entsprechende Draufsicht.

Figur 17a zeigt das Anschlusselement in einer Frontalansicht, wenn sich alle Segmente 5, 6 in einer vertikalen Lage H befinden. Figur 17b zeigt die entsprechende Draufsicht.

Bei einem Anschlusselement 2 gemäß den Figuren 15a und 15b wird vor dem Einbringen des Anschlusselements 2 in den Träger 1 zumindest ein erstes Segment 5 der zumindest zwei Segmente 5,6 in eine vertikale Lage V überführt und nach dem Einbringen des Anschlusselements 2 in den Träger 1 das zumindest eine erste Segment 5 wieder in eine horizontale Lage H überführt.

Bei einem Ausführungsbeispiel eines Anschlusselements 2 gemäß den Figuren 17a und 17b wird vor dem Einbringen des Anschlusselements 2 in den Träger 1 zumindest ein erstes Segment 5 in eine horizontale Lage H überführt und nach dem Einbringen des Anschlusselements 2 in den Träger 1 zumindest ein zweites Segment 6 in eine horizontale Lage H überführt wird.

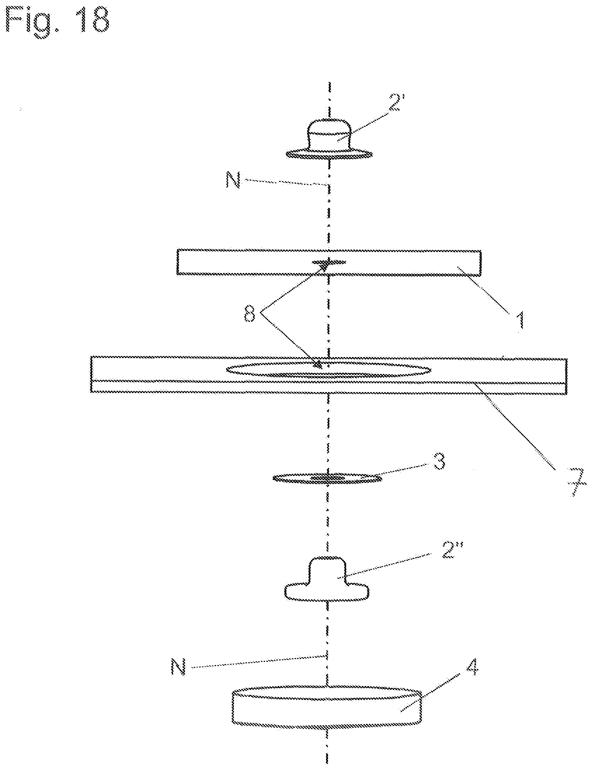

Bei dem in Figur 18 dargestellten weiteren Ausführungsbeispiel handelt es sich um eine Zentralelektrode mit zweiteiligem Anschlusselement. Die beiden Teile des Anschlusselementes 2 sind mit 2` und 2" bezeichnet. Zur Montage werden diese beiden Teile 2', 2" des Anschlusselementes 2 von verschiedenen Seiten in die Öffnung 8, Träger 1 und Leiter 3 eingebracht und zusammengesteckt. Sie können einfach klemmend miteinander verbunden sein. Es besteht aber auch die Möglichkeit, diese beiden Teile 2', 2" auch andersartig zu verbinden - beispielsweise durch Zusammenklemmen des Halses, durch Verkleben, Verschweißen oder Verlöten.

In Figur 18 ist weiters eine auf den Träger 1 verlaufende, gedachte Normale N eingezeichnet, die vorzugsweise zentral oder zentrisch durch das Anschlusselement 2 verläuft. Diese gedachte Normale N verläuft auch vorzugsweise zentrisch durch den ringförmig ausgebildeten Leiter 3 (genauer gesagt: durch die Öffnung des Leiter-Ringes) sowie durch das Kontaktmedium 4, dort auch vorzugsweise zentral. Bei dieser Zentralelektrode liegen also die Teile Anschlusselement 2, Leiter 3 und Kontaktmedium 4 direkt untereinander und sind nicht gegeneinander seitlich versetzt, vorzugsweise überhaupt nicht so wie in Figur 18 oder nur geringfügig, sodass immer noch die Normale N durch alle drei Teile 2, 3 und 4 (oder Öffnungen darin) verläuft oder diese schneidet.

Bei rotationssymmetrischen Komponenten bedeutet "zentrisch" durch den Mittelpunkt verlaufend. Bei nicht rotationssymmetrischen Komponenten bedeutet "zentrisch" durch den Schwerpunkt der Draufsichtsfläche verlaufend.

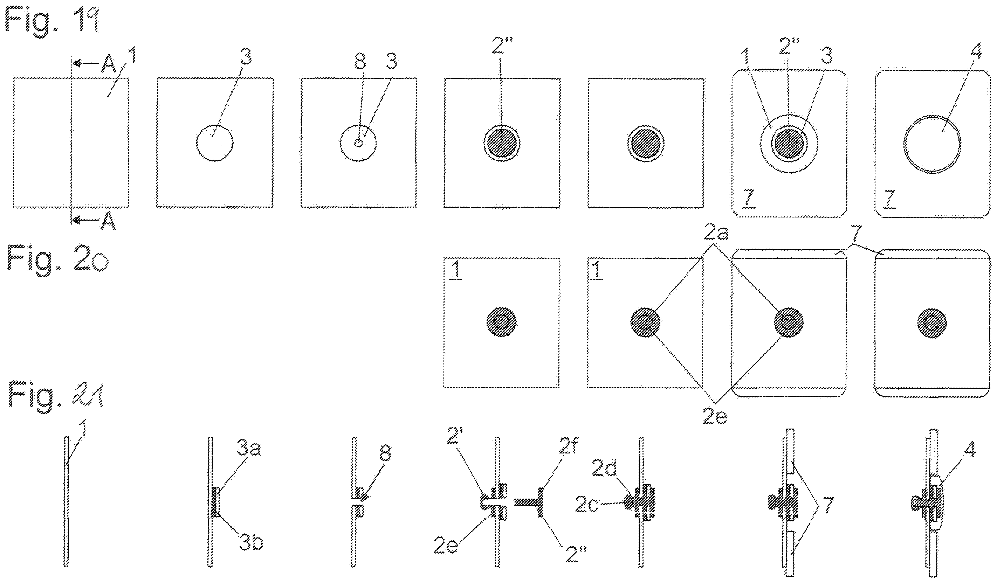

Bei dem in den Figuren 19 bis 21 dargestellten Ausführungsbeispiel stimmen die meisten Verfahrensschritte mit denen in Figur 1 bis 3 überein, weshalb auch gleiche Bezugszeichen gleiche Teile bezeichnen.

Der Unterschied besteht im Wesentlichen darin, dass ein zweiteiliges Anschlusselement 2 vorgesehen wird. Es werden ein erster Teil 2` und ein zweiter Teil 2" von unterschiedlichen Seiten des Trägers 1 herangeführt und zusammengesteckt.

Ein unterer Haltebereich 2f des zweiten Teils 2" des Anschlusselements 2 liegt dann am Träger 1oder am Leiter 3 an und verdeckt somit die später einem Kontaktmedium 4 zugewandte Seite des Leiters 3. Außerdem wird dadurch die galvanische Verbindung zwischen dem Kontaktelement 2 und dem Leiter 3 hergestellt. Dieser untere Haltebereich 2f entspricht funktionell im Wesentlichen dem verformten, erweiterten Bereich BZ der vorhergehenden Ausführungsbeispiele.

### Bezugszeichenliste:

- 1: Träger
- 2: Anschlusselement
2' erster Teil des Anschlusselementes 2
2" zweiter Teil des Anschlusselementes 2
- 2a: Anschlussstelle
- 2b: Vorsprung
- 2c: Kopf
- 2d: Hals
- 2e: Haltebereich
- 2f: unterer Haltebereich
- 3: Leiter
- 3a: elektrisch leitendes Material
- 3b: elektrisch nicht-leitendes Material
- 4: Kontaktmedium
- 5: erstes Segment
- 6: zweites Segment
- 7: Pflasterschicht
- 8: Öffnung
- 9: Flügelsegment
- 9a: Flügelsegmentabschnitt
- 11: Klebeschicht (Hautkleber)
- 11a: Ausnehmung
- H: Horizontale
- N: Normale
- V: Vertikale
- BZ: verformter, erweiterter Bereich

## Patentansprüche

1. Elektrode zum Anbringen auf der menschlichen Haut mit einem elektrisch nichtleitenden Träger (1), wobei auf einer der Haut abgewandten Oberseite des Trägers (1) ein vorstehendes, elektrisch leitendes Anschlusselement (2) mit einer Anschlussstelle (2a) zum lösbaren Anschließen eines Signalleiters vorgesehen ist, wobei ein zumindest teilweise auf der gegenüberliegenden Unterseite des Trägers (1) angeordneter Leiter (3) vorgesehen ist, welcher elektrisch mit dem Anschlusselement (2) sowie mit einem der Haut zugewandten Kontaktmedium (4) in Verbindung steht, wobei der Leiter (3) und das Kontaktmedium (4) auf der Unterseite des Trägers (1) unterhalb der Anschlussstelle (2a) des Anschlusselements (2) angeordnet sind, wobei eine durch das Anschlusselement (2) verlaufende, gedachte Normale auf den Träger (1) durch den Leiter (3) oder eine Ausnehmung (8) im Leiter (3) und das Kontaktmedium (4) oder eine Ausnehmung im Kontaktmedium (4) verläuft, wobei der Leiter (3) an seiner Oberfläche - vorzugsweise an der der Haut zugewandten Oberfläche - zumindest bereichsweise Silber / Silberchlorid oder Zinn / Zinnchlorid oder ein anderes beispielsweise zur Depolarisation der Elektrode geeignetes Redoxpaar aufweist, wobei das Anschlusselement (2) zumindest einen durch den Träger (1) reichenden Vorsprung (2b) aufweist, der an seinem Ende einen erweiterten Bereich (BZ) aufweist, und der Leiter (3) zumindest teilweise zwischen dem erweiterten Bereich (BZ) und dem Träger (1) angeordnet ist, wobei das Anschlusselement (2) an seiner Oberfläche frei von Silber oder Silber/Silberchlorid, und frei von Zinn oder Zinn/Zinnchlorid und frei von einem anderen - beispielsweise zur Depolarisation einer Elektrode geeigneten - Redoxpaar oder einer dessen Komponenten ist, und wobei das Anschlusselement (2) auf der Unterseite und/oder der Oberseite des Trägers (1) mit diesem unter Zwischenschaltung eines flächigen ausgebildeten Leiters (3) verbunden ist, und wobei die dem Kontaktmedium (4) zugewandte Seite des Leiters (3) teilweise oder vollständig von dem Anschlusselement (2) verdeckt wird.

2. Elektrode nach Anspruch 1, **dadurch gekennzeichnet, dass** der Silber / Silberchlorid oder Zinn / Zinnchlorid oder anderes zur Depolarisation der Elektrode geeignetes Redoxpaar aufweisende Bereich des Leiters (3) mit dem Anschlusselement (2) und/oder mit dem Kontaktmedium (4) elektrisch in Kontakt steht.

3. Elektrode nach einem der Ansprüche 1 oder 2, **dadurch gekennzeichnet, dass** der Leiter (3) als Schicht aus einem ersten Material (3b) ausgebildet ist, die im Bereich des Kontaktmediums (4) mit einem zweiten elektrisch leitenden Material (3a) versehen, vorzugsweise beschichtet, ist.

4. Elektrode nach Anspruch 3, **dadurch gekennzeichnet, dass** das zweite Material (3a) ringförmig auf dem ersten Material (3b) angeordnet ist, oder dass das zweite Material (3a) im Wesentlichen vollflächig auf dem ersten Material (3b) angeordnet ist.

5. Elektrode nach Anspruch 3 oder 4, **dadurch gekennzeichnet, dass** das erste Material (3b) ein Kunststoff, insbesondere eine Kunststofffolie, ist oder ein Leiter, beispielsweise aus Stahl ist, wobei das zweite Material (3a) vorzugsweise Silber / Silberchlorid, Zinn / Zinnchlorid oder ein anderes zur Depolarisation der Elektrode geeignetes Redoxpaar ist.

6. Elektrode nach einem der Ansprüche 1 bis 5, **dadurch gekennzeichnet, dass** der Leiter (3) durch eine auf den Träger (1) aufgebrachte, vorzugsweise aufgedruckte, Schicht aus einem leitfähigen Material gebildet wird.

7. Elektrode nach einem der Ansprüche 1 bis 6, **dadurch gekennzeichnet, dass** der Leiter (3) im Wesentlichen rotationssymmetrisch, insbesondere ringförmig, ausgebildet ist, oder dass der Leiter (3) im Wesentlichen quaderförmig ausgebildet ist.

8. Elektrode nach einem der Ansprüche 1 bis 7, **dadurch gekennzeichnet, dass** das Anschlusselement (2) aus zumindest zwei Teilen (2', 2") besteht, wobei einer der beiden Teile (2') die Anschlussstelle (2a) zum lösbaren Anschließen eines Signalleiters aufweist, oder dass das Anschlusselement (2) aus einem einzigen Teil besteht, das die Anschlussstelle (2a) zum lösbaren Anschließen eines Signalleiters aufweist.

9. Elektrode nach einem der Ansprüche 1 bis 8, **dadurch gekennzeichnet, dass** der erweiterte Bereich (BZ) durch Verformung gebildeten wird, wobei durch diesen verformten, erweiterten Bereich (BZ) einerseits eine elektrische Verbindung zwischen dem Leiter (3) und dem Anschlusselement (2a) und andererseits eine mechanische Befestigung des Anschlusselements (2) am Träger (1) herstellbar sind.

10. Elektrode nach einem der Ansprüche 1 bis 9, **dadurch gekennzeichnet, dass** die gedachte Normale auf den Träger (1) zentrisch durch das Anschlusselement (2) und ebenfalls zentrisch durch den Leiter (3) oder eine Ausnehmung (8) im Leiter (3) und das Kontaktmedium (4) oder eine Ausnehmung im Kontaktmedium (4) verläuft.

11. Elektrode nach einem der Ansprüche 1 bis 10, **dadurch gekennzeichnet, dass** der Träger (1) auf der der Haut zugewandten Seite mit Klebstoff, vorzugsweise einem Hautkleber (11), beschichtet ist, der vorzugsweise selbstklebend oder thermoaktivierbar ausgeführt ist, oder eine mit einem Klebstoff, vorzugsweise einem Hautkleber, versehene Pflasterschicht (7) aufweist.

## Claims

1. An electrode for application to the human skin having an electrically non-conducting carrier (1), wherein provided on a top side of the carrier (1) that faces away from the skin is a projecting electrically conducting connecting element (2) having a connecting location (2a) for releasable connection of a signal conductor, wherein there is provided a conductor (3) which is arranged at least partially on the opposite underside of the carrier (1) and which is electrically connected to the connecting element (2) and to a contact medium (4) that faces towards the skin, wherein the conductor (3) and the contact medium (4) are arranged on the underside of the carrier (1) beneath the connecting location (2a) of the connecting element (2), wherein a notional normal to the carrier (1) which extends through the connecting element (2) runs through the conductor (3) or an opening (8) in the conductor (3) and the contact medium (4) or an opening in the contact medium (4), wherein the conductor (3) at its surface - preferably at the surface facing towards the skin - at least region-wise has silver/silver chloride or tin/tin chloride or another redox couple which is suitable for example for depolarization of the electrode, wherein the connecting element (2) has at least one projection (2b) which extends through the carrier (1) and which at its end has an enlarged region (BZ) and the conductor (3) is arranged at least partially between the enlarged region (BZ) and the carrier (1), wherein the connecting element (2) at its surface is free from silver or silver/silver chloride and free from tin or tin/tin chloride and free from another redox couple - which is preferably suitable for depolarization of an electrode - or one of its components, and wherein the connecting element (2) is connected on the underside and/or the top side of the carrier (1) to same - preferably with the interposition of a conductor (3) of a planar configuration, and wherein the side of the conductor (3) facing the contact medium (4) is partially or completely covered by the connecting element (2).

2. Electrode according to claim 1, **characterized in that** the region of the conductor (3) having silver/silver chloride or tin/tin chloride or other redox couple suitable for depolarization of the electrode is electrically in contact with the connecting element (2) and/or with the contact medium (4).

3. Electrode according to one of claims 1 or 2, **characterized in that** the conductor (3) is in the form of a layer comprising a first material (3b) which is provided, preferably coated, with a second electrically conducting material (3a) in the region of the contact medium (4).

4. Electrode according to claim 3, **characterized in that** the second material (3a) is arranged in ring form on the first material (3b), or that the second material (3a) is arranged substantially over the full surface area on the first material (3b).

5. Electrode according to claim 3 or 4, **characterized in that** the first material (3b) is a plastic, in particular a plastic film, or a conductor, for example of steel, wherein the second material (3a) is preferably silver/silver chloride, tin/tin chloride or another redox couple suitable for depolarization of the electrode.

6. Electrode according to one of claims 1 to 5, **characterized in that** the conductor (3) is formed by a layer of a conductive material that is applied to, preferably printed on, the carrier (1).

7. Electrode according to one of claims 1 to 6, **characterized in that** the conductor (3) is of a substantially rotationally symmetrical, in particular ring-shaped, configuration, or that the conductor (3) is of a substantially cuboidal configuration.

8. Electrode according to one of claims 1 to 7, **characterized in that** the connecting element (2) comprises at least two parts (2', 2''), wherein one of the two parts (2') has the connecting location (2a) for releasably connecting a signal conductor, or that the connecting element (2) comprises a single part which has the connecting location (2a) for releasably connecting a signal conductor.

9. Electrode according to one of claims 1 to 8, **characterized in that** the enlarged region (BZ) is formed by deformation, wherein on the one hand an electrical connection between the conductor (3) and the connecting element (2a) and on the other hand a mechanical fixing of the connecting element (2) to the carrier (1) can be made by that deformed enlarged region (BZ).

10. Electrode according to one of claims 1 to 9, **characterized in that** the notional normal to the carrier (1) passes centrally through the connecting element (2) and also centrally through the conductor (3) or an opening (8) in the conductor (3) and the contact medium (4) or an opening in the contact medium (4).

11. Electrode according to one of claims 1 to 10, **characterized in that** the carrier (1) is coated on the side facing the skin with adhesive, preferably a skin adhesive (11), which is preferably a pressure sensitive adhesive or is thermo-activatable, or has a plaster layer (7) provided with an adhesive, preferably a skin adhesive.

## Revendications

1. Électrode destinée à être appliquée sur la peau humaine, comprenant un support (1) électriquement non conducteur, un élément de raccordement (2) saillant et électriquement conducteur étant prévu sur une face supérieure du support (1) opposée à la peau, lequel élément de raccordement comporte un point de raccordement (2a) pour le raccordement détachable d'un conducteur de signal, un conducteur (3) étant prévu, lequel conducteur est disposé au moins partiellement sur la face inférieure opposée du support (1), et est électriquement relié à l'élément de raccordement (2) ainsi qu'à un milieu de contact (4) orienté vers la peau, le conducteur (3) et le milieu de contact (4) étant disposés sur la face inférieure du support (1) en dessous du point de raccordement (2a) de l'élément de raccordement (2), une normale imaginaire au support (1) traversant l'élément de raccordement (2) passant à travers le conducteur (3), ou un évidement (8) dans le conducteur (3), et le milieu de contact (4), ou un évidement dans le milieu de contact (4), le conducteur (3) présentant sur sa surface - de préférence sur la surface orientée vers la peau - au moins localement de l'argent/chlorure d'argent ou de l'étain/chlorure d'étain ou un autre couple rédox approprié, par exemple pour la dépolarisation de l'électrode, l'élément de raccordement (2) présentant au moins une saillie (2b) traversant le support (1), laquelle comporte à son extrémité une zone élargie (BZ), et le conducteur (3) étant disposé au moins partiellement entre la zone élargie (BZ) et le support (1), l'élément de raccordement (2) étant dépourvu, à sa surface, d'argent ou d'argent/chlorure d'argent, d'étain ou d'étain/chlorure d'étain, ainsi que d'un autre couple rédox - approprié par exemple pour la dépolarisation d'une électrode - ou d'un de ses composants, et l'élément de raccordement (2) étant relié au support (1) sur la face inférieure et/ou supérieure de celui-ci, avec interposition d'un conducteur (3) de forme plane, et la face du conducteur (3) orientée vers le milieu de contact (4) étant partiellement ou totalement recouverte par l'élément de raccordement (2).

2. Électrode selon la revendication 1, **caractérisée en ce que** la zone du conducteur (3) comportant de l'argent/chlorure d'argent, de l'étain/chlorure d'étain ou un autre couple rédox approprié pour la dépolarisation de l'électrode est en contact électrique avec l'élément de raccordement (2) et/ou avec le milieu de contact (4).

3. Électrode selon l'une des revendications 1 ou 2, **caractérisée en ce que** le conducteur (3) est formé sous la forme d'une couche d'un premier matériau (3b), qui est pourvue, dans la zone du milieu de contact (4), d'un second matériau électriquement conducteur (3a), de préférence sous la forme d'un revêtement.

4. Électrode selon la revendication 3, **caractérisée en ce que** le second matériau (3a) est disposé de manière annulaire sur le premier matériau (3b), ou **en ce que** le second matériau (3a) est disposé sensiblement sur toute la surface du premier matériau (3b).

5. Électrode selon la revendication 3 ou 4, **caractérisée en ce que** le premier matériau (3b) est un matériau plastique, notamment un film plastique, ou un conducteur, par exemple en acier, le second matériau (3a) étant de préférence de l'argent/chlorure d'argent, de l'étain/chlorure d'étain ou un autre couple rédox approprié pour la dépolarisation de l'électrode.

6. Électrode selon l'une des revendications 1 à 5, **caractérisée en ce que** le conducteur (3) est formé par une couche d'un matériau conducteur appliquée sur le support (1), de préférence imprimée.

7. Électrode selon l'une des revendications 1 à 6, **caractérisée en ce que** le conducteur (3) est sensiblement de symétrie de rotation, notamment annulaire, ou **en ce que** le conducteur (3) est sensiblement de forme parallélépipédique.

8. Électrode selon l'une des revendications 1 à 7, **caractérisée en ce que** l'élément de raccordement (2) est constitué d'au moins deux parties (2', 2"), l'une des deux parties (2') comportant le point de raccordement (2a) pour le raccordement détachable d'un conducteur de signal, ou **en ce que** l'élément de raccordement (2) est constitué d'une seule pièce comportant le point de raccordement (2a) pour le raccordement détachable d'un conducteur de signal.

9. Électrode selon l'une des revendications 1 à 8, **caractérisée en ce que** la zone élargie (BZ) est obtenue par déformation, cette zone élargie déformée permettant d'une part d'établir une connexion électrique entre le conducteur (3) et l'élément de raccordement (2), et d'autre part d'assurer une fixation mécanique de l'élément de raccordement (2) sur le support (1).

10. Électrode selon l'une des revendications 1 à 9, **caractérisée en ce que** la normale imaginaire au support (1) passe de manière centrée à travers l'élément de raccordement (2) et également de manière centrée à travers le conducteur (3), ou un évidement (8) dans le conducteur (3), et le milieu de contact (4), ou un évidement dans le milieu de contact (4).

11. Électrode selon l'une des revendications 1 à 10, **caractérisée en ce que** le support (1) est revêtu, sur la face orientée vers la peau, d'un adhésif, de préférence un adhésif cutané (11), qui est de préférence auto-adhésif ou activable thermiquement, ou comporte une couche de type pansement (7) munie d'un adhésif, de préférence un adhésif cutané.
